(19) Europäisches Patentamt
European Patent Office
Office européen des brevets

(11) Veröffentlichungsnummer: **0 045 837**
**B1**

(12) # EUROPÄISCHE PATENTSCHRIFT

(45) Veröffentlichungstag der Patentschrift: **19.09.84**

(21) Anmeldenummer: **81104248.0**

(22) Anmeldetag: **03.06.81**

(51) Int. Cl.[3]: **C 07 D 311/94,** C 07 D 407/12 // C07H17/04, C07C177/00, (C07D407/12, 311/94, 309/12)

(54) **Reaktive Derivate des 3-Oxa-bicyclo(4.3.0)-non-1-en-9-ons, Verfahren zu ihrer Herstellung und deren Verwendung.**

(30) Priorität: **12.08.80 DE 3030477**

(43) Veröffentlichungstag der Anmeldung: **17.02.82 Patentblatt 82/7**

(45) Bekanntmachung des Hinweises auf die Patenterteilung: **19.09.84 Patentblatt 84/38**

(84) Benannte Vertragsstaaten: **AT BE CH FR GB IT LI NL SE**

(56) Entgegenhaltungen:
**DE - A - 1 768 069**
**US - A - 3 089 877**

**CHEMICAL ABSTRACTS, vol. 82, no. 23, 9. Juni 1975, Columbus, Ohio, USA BIANCO et al. "Iridoids. XVI. Structure and configuration of mioporoside, a new glucoside", page 646, column 1, abstract 156641p**

(73) Patentinhaber: **Dr. Willmar Schwabe GmbH & Co., Willmar-Schwabe-Strasse 4, D-7500 Karlsruhe 41 (DE)**

(72) Erfinder: **Weinges, Klaus, Dr. Prof. Dipl.-Chem., Langgewann 41, D-6900 Heidelberg (DE)**
Erfinder: **von der Eltz, Herbert, Dipl. Bio. Dipl. Chem., Beethovenstrasse 14, D-6831 Plankstadt (DE)**
Erfinder: **Jaggy, Hermann, Dr. Dipl.-Chem., Kapellenweg 7, D-7525 Bad Schönborn 1 (DE)**

(74) Vertreter: **Bunke, Max, Dipl.-Ing. et al, Patentanwälte Prinz, Bunke & Partner Lessingstrasse 9, D-7000 Stuttgart 1 (DE)**

**EP 0 045 837 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft reaktive Derivate des 3-Oxa-bicyclo[4.3.0]-non-1-en-9-ons der allgemeinen Formel I

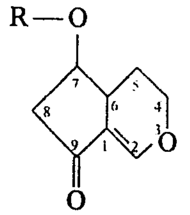

(I)

worin R ein Wasserstoffatom, eine Alkylgruppe mit 1 bis 5 C-Atomen, eine Acylgruppe mit 2 bis 6 C-Atomen, eine unsubstituierte Aralkylgruppe mit 7 bis 12 C-Atomen, eine Methansulfonyl- oder Toluolsulfonylgruppe, eine Benzoyl-, Nitrobenzoyl- oder Chlorbenzoylgruppe oder eine Tetrahydropyranyl-Gruppe bedeutet.

Verbindungen der allgemeinen Formel I werden auch als »Iridoide« bezeichnet.

Die Iridoide sind eine Gruppe von Naturstoffen, deren gemeinsames Strukturmerkmal das Cyclopentanpyran-Ringsystem ist:

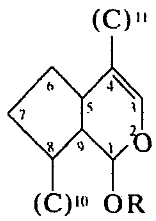

Die in der Natur vorkommenden Iridoide liegen meist in Form von Glycosiden vor, wobei der Zucker mit dem $C^1$-Atom des Iridoids verknüpft ist. Ein aus der Droge Picrorhiza kurrooa leicht isolierbares Iridoidglucosid ist Catalpol der Formel II,

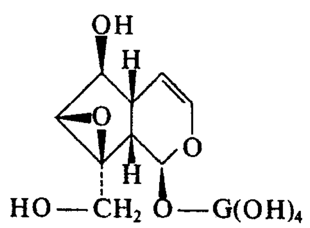

(II)

($-O-G(OH)_4$ = 1-β-D-Glucopyranosyl-Rest)

das durch den Epoxidring zwischen $C^7$ und $C^8$ gekennzeichnet ist und als 1-β-D-glucopyranosid vorliegt (vgl. V. Plouvier, Compt. Rend. 224, 670 (1947).

Eine Übersicht über die Iridoidglucoside und ihre Isolierung geben O. Sticher und U. Junod-Busch in Pharm. Acta Helv. 50, S. 127–144 (1975).

Der Erfindung liegt die Aufgabe zugrunde, die Verbindungen der allgemeinen Formel I bereitzustellen und ein möglichst einfaches Verfahren zu ihrer Herstellung und damit einen neuen und einfachen Zugang zu reaktiven Iridoid-Derivaten zu schaffen, um so neue Wege zur Synthese pharmakologisch wirksamer Naturstoffklassen, insbesondere der Prostanoide, zu eröffnen.

Diese Aufgabe wird durch die Bereitstellung der erfindungsgemäßen Verbindungen, das erfindungsgemäße Verfahren und die erfindungsgemäße Verwendung dieser Verbindungen gelöst.

Besonders bevorzugte erfindungsgemäße Verbindungen sind 7-Hydroxy-3-oxa-bicyclo[4.3.0]non-1-en-9-on und 7-Acetoxy-3-oxa-bicyclo[4.3.0]non-1-en-9-on, die aufgrund ihrer asymmetrischen Kohlenstoffatome $C^6$ und $C^7$ in Form ihrer optisch aktiven (+)- und (−)-Diastereomeren oder in Form ihrer Racemate vorliegen können.

Bei der Nomenklatur der erfindungsgemäßen Verbindungen ist darauf zu achten, daß sich die Bezifferung des Ringsystems unterscheidet, je nachdem, ob es als Iridoid-Derivat oder als Bicyclo[4.3.0]nonenon bezeichnet wird:

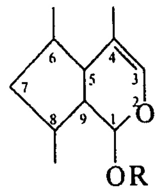

Iridoid-Bezifferung

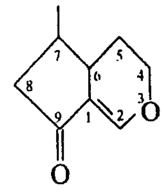

Bicyclo[4.3.0]nonenon-Bezifferung

Die erfindungsgemäßen Verbindungen der allgemeinen Formel I lassen sich in vier oder fünf Reaktionsstufen ohne zeitraubende Trennungen aus natürlichem Catalpol (1a), und zwar nach dem folgenden Reaktionsschema, herstellen:

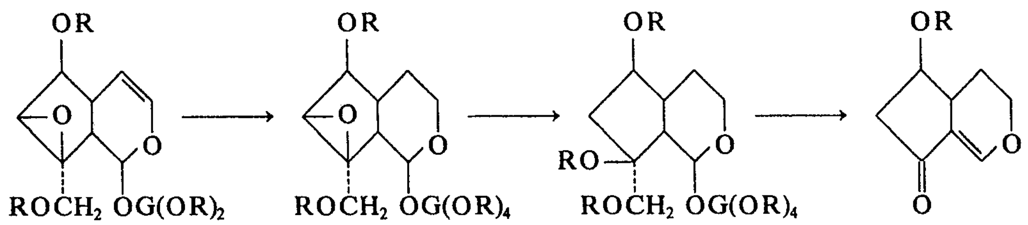

| | | | |
|---|---|---|---|
| (1a): R = H | (2a): R = H | (3a): R = H | (4a): R = H |
| (1b): R = $CH_3CO$ bzw. Acyl- | (2b): R = $CH_3CO$ bzw. Acyl- | (3b): R = $CH_3CO$ bzw. Acyl- | (4b): R = $CH_3CO$ bzw. Acyl- |

In der ersten Reaktionsstufe wird Catalpol mit dem Anhydrid einer Carbonsäure, welche 2 bis 6 C-Atome aufweist, beispielsweise mit Acetanhydrid, in einem geeigneten Lösungsmittel, vorzugsweise in wasserfreiem Pyridin bei Raumtemperatur zum Hexa-acyl-catalpol (1b) acyliert (O-Acylierung). Dabei werden die vier freien Hydroxylgruppen der β-D-Glucopyranose ebenfalls O-acyliert.

In der zweiten Reaktionsstufe wird das entstandene Hexaacyl-catalpol (1b) durch katalytische Hydrierung quantitativ in die entsprechende gesättigte Verbindung, nämlich das Hexaacyl-dihydrocatalpol (2b) übergeführt.

Das Hexaacyl-dihydrocatalpol (2b) wird in der dritten Reaktionsstufe durch Umsetzung mit Lithiumalanat in einem dipolaren aprotischen Lösungsmittel, vorzugsweise in absolutem Tetrahydrofuran, umgesetzt, wodurch der Epoxid-Ring regioselektiv aufgespalten wird und sämtliche Acylreste wieder abgespalten werden, so daß das 6,8-Dihydroxy-8-hydroxymethyl-1-iridanyl-1'-β-D-glucopyranosid (3a) entsteht.

In der vierten Reaktionsstufe wird dann die Verbindung (3a) mit Perjodsäure oder einem ihrer Salze, insbesondere mit Natriumperjodat, oder mit Bleitetraacetat, vorzugsweise in wäßriger Lösung einer Glykolspaltung unterzogen, wonach die Reaktionslösung mit einer schwachen Base, beispielsweise wäßriger Hydrogencarbonatlösung, gesättigt wird. Der letzte Verfahrensschritt ist insofern bemerkenswert, als durch die Oxidation mit Natriumperjodat bei den Iridoidglucosiden normalerweise die Glucosid-Bindung — im Gegensatz zu anderen Glucosiden — gespalten wird, so daß neben den aus der Glucose entstehenden Carbonsäuren die Aglyka erhalten werden. Dies hat gegenüber der bekannten säurekatalysierten Glucosid-Spaltung den Vorteil, daß die Aglyka direkt nach der Sättigung mit einer geeigneten Base aus der schwach alkalischen Lösung isoliert werden können und nicht von der sonst entstehenden Glucose abgetrennt werden müssen. Überraschenderweise erhält man aber bei der oxidativen Spaltung der Verbindung (3a) nicht das Aglykon, sondern direkt das erfindungsgemäße (6R, 7R)-(—)-7-Hydroxy-3-oxa-bicyclo[4.3.0]non-1-en-9-on (4a), weil hier nach der Spaltung der Glucosid-Bindung in schwach alkalischem Medium gleichzeitig Wasser abgespalten wird.

In der fünften Reaktionsstufe wird die Verbindung (4a) mit Hilfe an sich bekannter Methoden alkyliert, acyliert oder kondensiert, um gegebenenfalls zu den gewünschten O-substituierten Derivaten der allgemeinen Formel I zu gelangen. Die Acylierung der 7-Hydroxyl-Gruppe der Verbindung (4a) wird mit dem entsprechenden Carbonsäureanhydrid oder Carbonsäurehalogenid durchgeführt, beispielsweise mit Acetanhydrid, Benzoylchlorid, p-Nitrobenzoylchlorid, Methansulfonylchlorid, Toluolsulfonylchlorid. Die Alkylierung wird in ganz entsprechender Weise mit einem Alkylhalogenid mit 1 bis 5 C-Atomen, beispielsweise Äthylbromid, mit einem Aralkylhalogenid, beispielsweise Benzylchlorid, mit einem Alkylsulfat oder Alkyl-p-toluolsulfonat durchgeführt. Die Kondensation kann mit jeder geeigneten Verbindung, z. B. mit Tetrahydropyran, durchgeführt werden.

Die erfindungsgemäßen Verbindungen können besonders vorteilhaft als reaktive Zwischenprodukte zur Synthese bzw. Partialsynthese von Naturstoffen, insbesondere von Prostanoiden, verwendet werden. Die Prostanoide oder Prostaglandine werden bekanntlich den Gewebshormonen zugerechnet und weisen ein breites pharmakologisches Wirkungsspektrum auf. Sie wirken besonders auf die glatte Muskulatur und auf Durchblutungsvorgänge, sind lokale Modulatoren hormonaler Wirkungen, stimulieren die Prolaktin-Sekretion, sie sind an der Blutstillung sowie der immunologischen Abwehr beteiligt. Alle Säugetierzellen können Prostaglandine synthetisieren. Sie werden durch viele physiologische, pharmakologische und pathologische Reize freigesetzt.

Eine kurze Zusammenfassung zur Chemie und zum Metabolismus der Prostaglandine gibt H. König in Klin. Wochenschrift 53, S. 1041 bis 1048 (1975).

Die erfindungsgemäßen Verbindungen eröffnen einen neuen und chemisch eigenartigen Syntheseweg zu den pharmakologisch äußerst wichtigen Prostanoiden, dessen erste Stufen sich aus dem folgenden Formelschema ergeben:

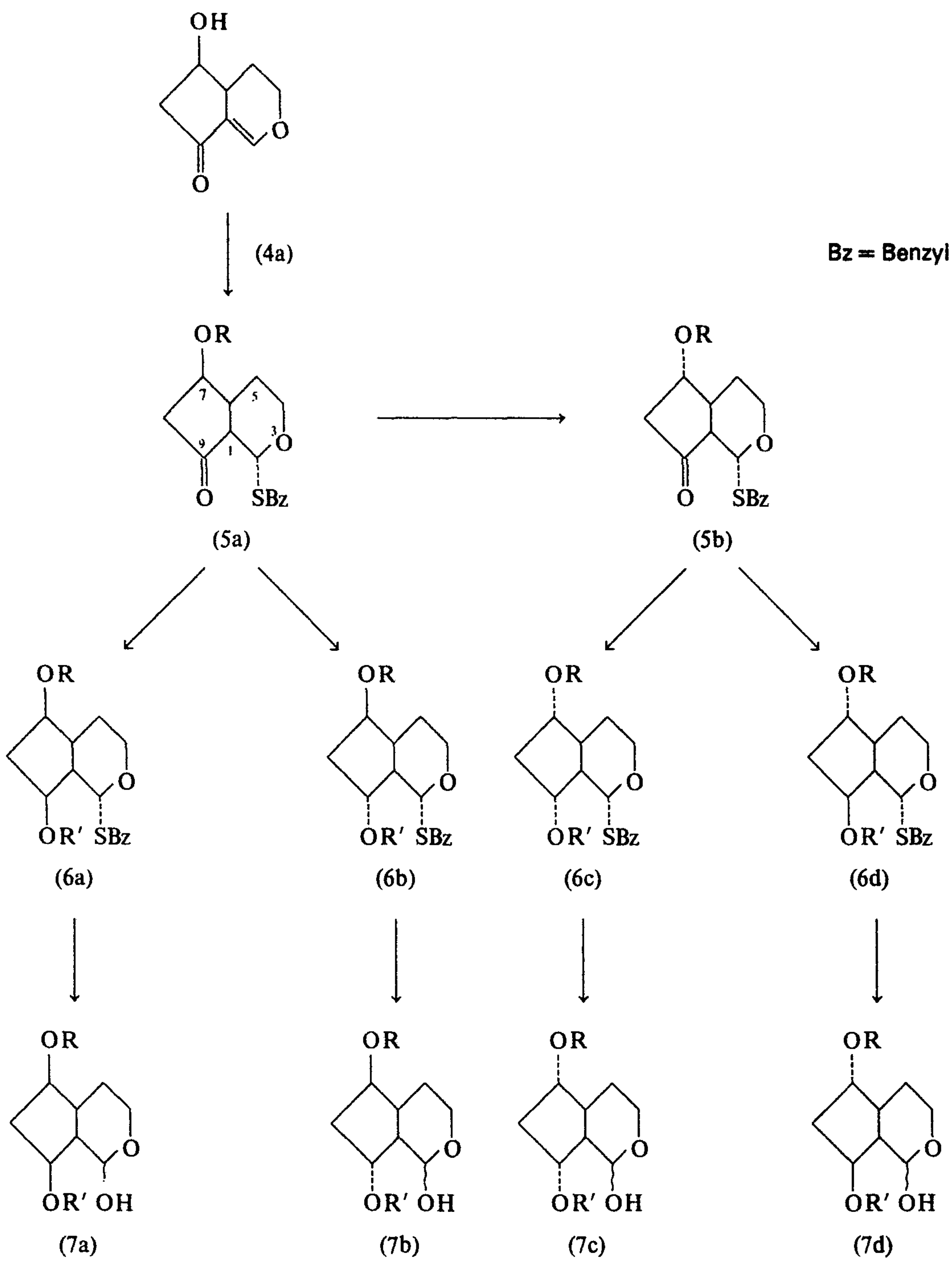

(4a)

Bz = Benzyl

(5a) (5b)

(6a) (6b) (6c) (6d)

(7a) (7b) (7c) (7d)

In der ersten Reaktionsstufe wird dabei an die Doppelbindung des Ketoenoläthers (4a) Benzylmercaptan oder ein anderes geeignetes Nucleophil, beispielsweise die N≡Cl -Gruppe, addiert. Die Addition von Benzylmercaptan führt zu 1-Benzylthio-7-hydroxy-3-oxa-bicyclo[4.3.0]nonan-9-on (5a). Vorteilhafterweise kann die Reaktionsstufe 4 zur Herstellung des Ketoenoläthers (4a) und die Benzylmercaptan-Addition in einem Zuge, direkt hintereinander, ausgeführt werden.

In der zweiten Reaktionsstufe wird die Verbindung (5a) mit Natriumboranat zu (7R, 9S)-(−)-1-Benzylthio-7,9-dihydroxy-3-oxa-bicyclo[4.3.0]nonan (6a) (Hauptprodukt) und zu dem (7R, 9R)-Diastereomeren (6b) (Nebenprodukt) reduziert. Die Diastereomeren werden säulenchromatographisch quantitativ getrennt. Die Reaktion kann auch stereoselektiv ausgeführt werden, so daß nur Verbindung (6a) entsteht (vgl. E. Martinez, J.M. Muchowski und E. Velade, J. Org. Chem. 42, 1087 (1977)).

Die Benzylthio-Gruppen der entstandenen Verbindungen lassen sich mit Quecksilberacetat quantitativ abspalten, wodurch die entsprechenden 2,7,9-Trihydroxy-3-oxa-bicyclo[4.3.0]nonane (7a) und (7b) entstehen.

Die stereoisomere Reihe der Verbindungen (5b), (6c), (6d), (7c) und (7d) erhält man durch Inversion der Hydroxylgruppe am $C^7$-Atom der Verbindung (5a), und zwar nach dem Verfahren von H. Loibner und E. Zbiral, Helv. Chim. Acta 59, 2100 (1976). Die weiteren Umsetzungen der Verbindung (5b) zu den Verbindungen (7c) und (7d) werden analog den oben beschriebenen Reaktionsstufen 2 und 3 durchgeführt.

Die weitere Umsetzung der Verbindungen (7a) bis (7d) zu den Prostanoiden erfolgt entsprechend dem folgenden Formelschema:

(7a-d)

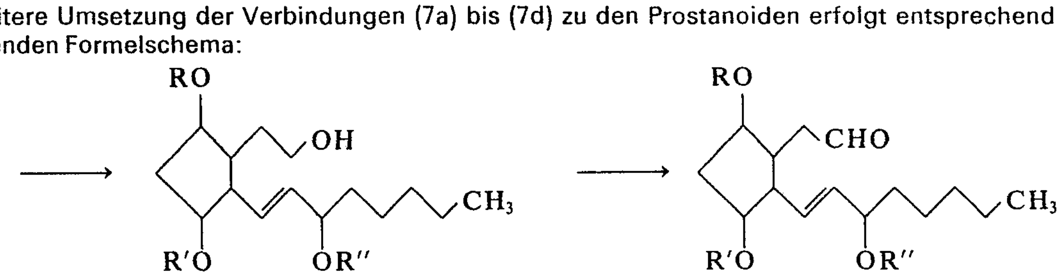

(8) (9)

(9)

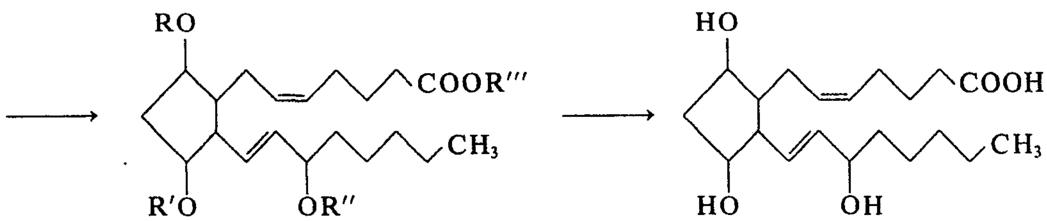

(10) (11)

Dabei werden die Halbacetale (7a) bis (7d) mit dem entsprechenden Wittig-Reagens, also mit entsprechend substituierten Monoalkyl-triphenyl-phosphonium-Salzen (vgl. E.J. Corey et al., J. Am. Chem. Soc. 93, 1490 (1971)) zur Verbindung (8) mit der durch (7a) bis (7d) vorgegebenen Konfiguration umgesetzt. Die bei der Spaltung des Pyran-Ringsystems entstandene primäre Hydroxylgruppe wird anschließend mit Pyridiniumdichromat (PDC) (vgl. Tetrahedron Lett. 1979, 399) oder mit Pyridiniumchlorochromat (PCC) (vgl. Tetrahedron Lett. 1975, 2647) zum Aldehyd (9) oxidiert. Die zweite Seitenkette des gewünschten Prostanoids wird wiederum durch eine Wittig-Reaktion an die Verbindung (9) angefügt (Wittig-Reagens: vgl. J.S. Bindra und R. Bindra, Prostaglandin Synthesis, Academic Press, Inc., New York, 1977, S. 210). Schließlich werden die Schutzgruppen R, R′, R″ und R‴, beispielsweise Acetyl-, Benzyl-, Benzoyl-, p-Nitrobenzoyl-, Mesyl-, Tosyl-Gruppen und ähnliche bekannte Schutzgruppen, in an sich bekannter Weise abgespalten.

Besonders bevorzugte Ausführungsformen der Erfindung ergeben sich aus den folgenden Beispielen.

## Beispiel 1

### Catalpol (1a) aus Picrorhiza kurrooa

10 kg gemahlene Droge von Picrorhiza kurrooa werden mit 100 kg 5%iger Sodalösung versetzt und durch Einleiten von Wasserdampf 3 h auf 93–95° C erhitzt. Durch das Einleiten des Dampfes wird die Droge aufgewirbelt und dadurch gut extrahiert. Über Nacht wird durch ein Perlontuch filtriert und der Rückstand nochmals mit 80 kg 5%iger Sodalösung extrahiert. Die vereinigten Filtrate werden 20 min bei Siedehitze gehalten und 3 h bei 80° C mit 10 kg Aktivkohle gerührt. Die Kohle läßt man über Nacht absitzen. Die dekantierte Lösung wird nochmals mit 4 kg Aktivkohle behandelt. Man saugt die vereinigte Kohle durch eine Steingutnutsche mit 60 cm Durchmesser, die zuvor mit einer Aufschlämmung von ca. 2 kg Hyflo-Super-Cel® belegt wird, ab und wäscht mit Wasser, bis das Filtrat ca. pH 8 zeigt. Die an der Luft getrocknete Kohle wird dreimal mit je 50 kg 95%igem Ethanol ausgekocht. Nach dem Absaugen erhält man ca. 166 kg Lösung, die in einer Destillationsanlage auf ca. 30 kg vorkonzentriert und dann in einem 100-l-Rotationsverdampfer bis zur Trockne eingeengt wird. Anschließende Gefriertrocknung ergibt ein dunkelbraunes Catalpol-Konzentrat, das noch größere Mengen Zucker und wenig Picrosid-Gemisch enthält. Ausbeute 1570 g (14,5% bez. auf die getrocknete Droge).

Zu 250 g des Catalpol-Konzentrats werden 300 g $Al_2O_3$ (neutral, Aktivitätsstufe I) und 1,5 l Ethanol gegeben. Unter Rühren wird zum Sieden erhitzt und 1 l Ethanol abdestilliert. Diese Mischung wird auf eine $Al_2O_3$-Säule (100 × 5 cm; 1200 g $Al_2O_3$ mit Ethanol gewaschen) aufgeschlämmt und mit Ethanol/Wasser (9 : 1) eluiert. Die Fraktionen, die das Catalpol enthalten, werden dünnschicht-chromatographisch ermittelt (DC: $R_F$ = 0,34; Fließmittel: $CHCl_3/CH_3OH/2$ N $CH_3COOH$ 70 : 30 : 6) und vereinigt. Die Lösung wird vollständig i. Vak. bei 52°C Badtemperatur zur Trockne eingedampft. Der zunächst anfallende Schaum kristallisiert aus Ethanol. Die Kristalle werden abgesaugt und mit Ethanol gut gewaschen. Durch erneute säulenchromatographische Trennung der Mutterlauge über eine $Al_2O_3$-Säule kann weiteres Catalpol isoliert werden.

Catalpol (1a), $C_{15}H_{22}O_{10}$ (362,3),
Ausbeute 45 g (18% bez. auf das Catalpol-Konzentrat), Schmp. 202—204°C, $[\alpha]^{20}_{589} = -39{,}7°$ (c = 1,2 g in 100 ml Ethanol).

1. Reaktionsstufe

15 g Catalpol (1a) werden bei Raumtemperatur in 24 ml absol. Pyridin gelöst und mit 30 ml Acetanhydrid versetzt. Die Reaktionslösung bleibt 15 h bei Raumtemperatur stehen und wird anschließend in Eiswasser gegossen. Das ausgefallene Produkt wird so lange geknetet, bis es fest ist und abgesaugt werden kann. Das amorphe Produkt wird mit Eiswasser gewaschen, getrocknet und aus wenig Ethanol umkristallisiert.

Hexaacetyl-catalpol (1b), $C_{27}H_{34}O_{16}$ (614,6),
Ausbeute 22 g (86%), Schmp. 142—143°C, $[\alpha]^{20}_{589} = -87{,}3°$ (c = 1 g in 100 ml $CHCl_3$), RF = 0,34 (Fließmittel: Benzol/Aceton 8 : 2).

2. Reaktionsstufe

22 g Hexaacetyl-catalpol (1b) werden in ca. 50 ml Essigester gelöst und mit 1,5 g Pd/C-Katalysator (10% Pd) versetzt. In einer Hydrierapparatur wird so lange hydriert, bis kein Wasserstoff mehr aufgenommen wird (in ca. 2 h ca. 970 ml $H_2$; ber. 802 ml $H_2$). Die Lösung wird vom Katalysator abfiltriert und das Filtrat bis zur Trockne i. Vak. (Badtemp. 45°C) eingedampft. Der Rückstand wird aus wenig Ethanol umkristallisiert.

Hexaacetyl-dihydrocatalpol (2b), $C_{27}H_{36}O_{16}$ (616,6),
Ausbeute 22 g (99%), Schmp. 155—156°C, $[\alpha]^{20}_{589} = -80{,}4°$ (c = 1 g in 100 ml $CHCl_3$), $R_F$ = 0,30 (Benzol/Aceton 8 : 2).

3. Reaktionsstufe

Zu einer Suspension von 7,4 g (195 mmol) $LiAlH_4$ in 1000 ml wasserfreiem Tetrahydrofuran (THF) werden portionsweise 18,4 g (30 mmol) Hexaacetyl-dihydrocatalpol (2b) gegeben und 4 h unter Rühren und Rückfluß gekocht. Das überschüssige $LiAlH_4$ wird mit Essigester und Wasser zersetzt. Nach Einleiten von $CO_2$ wird von den anorganischen Salzen abfiltriert und der Rückstand mehrmals mit Wasser gewaschen. Das THF wird i. Vak. bei 40°C Badtemperatur abgedampft und die wäßrige Lösung mit 50 g Aktivkohle 3 h auf 80°C erwärmt (Rührer). Nach dem Dekantieren von der abgesetzten Kohle wird die Lösung noch zweimal mit je 50 g Aktivkohle behandelt. Das DC zeigt, daß kein 3a mehr in der wäßrigen Lösung vorhanden ist. Die Kohle wird abgesaugt und mit Wasser so lange gewaschen, bis keine anorganischen Salze mehr nachzuweisen sind. Nach dem Trocknen der Kohle an der Luft wird sie mehrmals mit 95%igem Ethanol 10 min in der Siedehitze extrahiert. Die gesammelten Filtrate werden i. Vak. eingedampft.

6,8-Dihydroxy-8-(hydroxymethyl)-1-iridanyl-1'-β-D-glucopyranosid (3a), $C_{15}H_{26}O_{10}$ (366,4),
Ausbeute 9,5 g (86%), amorph, $[\alpha]^{20}_{589} = -77{,}1°$ (c = 1,9 g in 100 ml $CH_3OH$), $R_F$ = 0,27 ($CHCl_3/CH_3OH/2$ N $CH_3COOH$ 60 : 50 : 6).

4. Reaktionsstufe

Zu einer Lösung von 5,5 g (15 mmol) 3a in 250 ml Wasser werden 15 g (70 mmol) Natriumperjodat gegeben. Die Lösung wird bei gelegentlichem Umschütteln $^1/_2$ h bei Raumtemperatur stehengelassen. Nach Zugabe von 20 g Natriumhydrogencarbonat (pH = 8) werden die anorganischen Salze abfiltriert und mit 50 ml Wasser gewaschen. Das Filtrat wird i. Vak. bei 35°C Badtemperatur so weit eingedampft, bis weitere anorganische Salze auszufallen beginnen. Die farblose Lösung wird fünfmal mit je 100 ml Essigester extrahiert. Der Essigester wird mit Natriumsulfat getrocknet und i. Vak. bei 35°C Badtemperatur vollständig eingedampft. Das zunächst anfallende Öl kristallisiert beim Anreiben. Zur Analyse wird das Produkt in sehr wenig kaltem Dioxan gelöst und bis zur Trübung mit Tetrachlorkohlenstoff versetzt. Im Kühlschrank kristallisiert 4a

(6R, 7R)-(−)-7-Hydroxy-3-oxa-bicyclo[4.3.0]non-1-en-9-on (4a), $C_8H_{10}O_3$ (154,2),
Ausbeute 1,8 g (78%), Schmp. 95—97°C, $[\alpha]^{20}_{589} = -267°$ (c = 3 g in 100 ml $CH_3OH$), $R_F = 0,32$ ($CHCl_3/CH_3OH$ 9 : 1).

Verwendung der erfindungsgemäßen Verbindungen zur Synthese von Prostanoiden

1. Reaktionsstufe

Zu einer Lösung von 1,6 g (10,4 mmol) Ketoenolether (4a) in 3 ml THF werden nacheinander 2 ml Benzylmercaptan und 0,2 ml Triethylamin gegeben. Man rührt bei Raumtemperatur 5 h. Die Reaktionslösung wird i. Vak. zur Trockne eingedampft. Der Rückstand wird aus Tetrachlorkohlenstoff umkristallisiert.

1-(Benzylthio)-7-hydroxy-3-oxa-bicyclo[4.3.0]nonan-on (5a), $C_{15}H_{18}O_3S$ (278,3),
Ausbeute 2,3 g (79,6%), Schmp. 118°C, $[\alpha]^{20}_{589} = -188°$ (c = 2 g in 100 ml $CHCl_3$), $R_F = 0,23$ (Benzol/Aceton 8 : 2).

2. Reaktionsstufe

Zu einer Lösung von 265 mg (7 mmol) $NaBH_4$ in 20 ml absol. Methanol wird bei —15° bis —20°C eine Lösung von 1,95 g (7 mmol) 5a in 20 ml absol. Methanol innerhalb von ca. $^1/_2$ h zugetropft. Man rührt 4 h bei —15°C. Das Kühlbad wird entfernt, und man läßt die Lösung unter Einleiten von Kohlendioxid auf Raumtemperatur erwärmen. Während des Einleitens tropft man 50 ml Wasser zu. Die Reaktionslösung wird fünfmal mit je 50 ml Ether ausgeschüttelt. Die Etherphase wird mit wasserfreiem Natriumsulfat getrocknet und i. Vak. zur Trockne eingedampft. Der Rückstand wird über eine Drucksäule (80 × 2 cm; Füllmaterial: Kieselsäure) nacheinander mit 300 ml Benzol, 1600 ml Benzol/Aceton (95 : 5), 1600 ml Benzol/Aceton (90 : 10) und 1000 ml Benzol/Aceton (80 : 20) eluiert. Die Fraktionen werden dünnschichtchromatographisch ermittelt (Fließmittel: Chloroform/Methanol 9 : 1).

(7R, 9S)-(−)-1-(Benzylthio)-7,9-dihydroxy-3-oxa-bicyclo[4.3.0]nonan (6a), $C_{15}H_{20}O_3S$ (280,3),
Ausbeute 1,13 g (57,6%). Öl, $[\alpha]^{20}_{589} = -340°$ (c = 1,6 g in 100 ml Aceton), $R_F = 0,38$ ($CHCl_3/CH_3OH$ 9 : 1).
(7R, 9R)-(−)-1-(Benzylthio)-7,9-dihydroxy-3-oxa-bicyclo[4.3.0]nonan (6b), $C_{15}H_{20}O_3S$ (280,3),
Ausbeute 295 mg (15%), Kristalle aus Tetrachlorkohlenstoff, Schmp. 78—79°C, $[\alpha] = -154,9°$ (c = 2 g in 100 ml Aceton), $R_F = 0,32$ ($CHCl_3/CH_3OH$ 9 : 1).

3. Reaktionsstufe

Zu einer Lösung von 175 mg (0,55 mmol) Quecksilberacetat in 3,5 ml Wasser wird innerhalb von 3 min eine Lösung von 280 mg (1 mmol) 6a bzw. 6b in 3,5 ml Acetonitril zugetropft. Man rührt 10 min und verdünnt mit 15 ml Wasser. Nach dem Filtrieren der Lösung wird sie i. Vak. zur Trockne eingedampft. Der Rückstand wird über eine Drucksäule (80 × 1 cm; Füllmaterial : Kieselsäure) mit Chloroform/Methanol (9 : 1) gereinigt.

(7R, 9S)-(−)-2,7,9-Trihydroxy-3-oxa-bicyclo[4.3.0]nonan (7a), $C_8H_{14}O_4$ (174,2),
Ausbeute 80%, Öl; der spezif. Drehwert wurde noch nicht bestimmt, $R_F = 0,24$ ($CHCl_3/CH_3OH$ 8 : 2).
(7R, 9R)-(−)-2,7,9-Trihydroxy-3-oxa-bicyclo[4.3.0]nonan (7b), $C_8H_{14}O_4$ (174,2),
Kristalle aus wenig Aceton, Ausbeute 84%, Schmp. 108—110°C, $[\alpha]^{20}_{589} = -21,7°$ (c = 1 g in 100 ml Methanol), $R_F = 0,22$ ($CHCl_3/CH_3OH$ 8 : 2).

4. Reaktionsstufe

1,3 g (4,7 mmol) 5a werden mit 3,7 g (14 mmol) Triphenylphosphin und 1,15 g (9,4 mmol) Benzoesäure in 60 ml absol. Benzol gelöst. Unter Rühren wird bei Raumtemperatur eine benzolische Lösung von 1,64 g (9,4 mmol) Azodicarbonsäurediethylester zugetropft (Tropfgeschwindigkeit: 1 Tr./3 sec). Während der Reaktion fällt ein weißer Niederschlag von Hydrazodicarbonsäurediethylester aus. Sobald eine schwache Gelbfärbung der Reaktionslösung auftritt, beendet man die Reaktion. Dünnschichtchromatographisch läßt sich kein Ausgangsmaterial mehr nachweisen. Der Niederschlag wird abgesaugt und das Filtrat auf Celite aufgezogen. Die Substanz wird über eine Drucksäule (80 × 2 cm; Füllmaterial: Kieselsäure) mit Benzol/Aceton (95 : 5) gereinigt.

(6R, 7S)-1-(Benzylthio)-7-(benzoyloxy)-3-oxa-bicyclo[4.3.0]nonan-9-on (5b; R = Benzoyl-), $C_{22}H_{22}O_4S$ (328,5), Ausbeute 1,3 g (56%), Öl. $R_F$ = 0,52 (Benzol/Aceton 8 : 2). Der $R_F$-Wert von 5b (R = Benzoyl-) unterscheidet sich von dem ($R_F$ = 0,64 im gleichen Fließmittel) von 5a (R = Benzoyl-). Weiter wurde das Produkt noch nicht untersucht. Durch Verseifung sollte man 5b (R = H) erhalten.

5.—8. Reaktionsstufe

Die Weiterverarbeitung der Verbindungen 7a, 7b, 7c und 7d zu den Prostanoiden 11 erfolgt, wie oben beschrieben (vgl. S. 8/9).

**Patentansprüche**

1. Reaktive Derivate des 3-oxa-bicyclo[4.3.0]-non-1-en-9-on der allgemeinen Formel I,

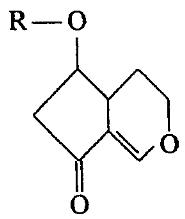

(I)

worin R ein Wasserstoffatom, eine Alkylgruppe mit 1 bis 5 C-Atomen, eine Acylgruppe mit 2 bis 6 C-Atomen, eine unsubstituierte Aralkylgruppe mit 7 bis 12 C-Atomen, eine Methansulfonyl- oder Toluolsulfonylgruppe, eine Benzoyl-, Nitrobenzoyl- oder Chlorbenzoyl- oder eine Tetrahydropyranylgruppe bedeutet.

2. 7-Hydroxy-3-oxa-bicyclo[4.3.0]non-1-en-9-on.

3. [6R, 7R)-(—)-7-Hydroxy-3-oxa-bicyclo[4.3.0]non-1-en-9-on.

4. 7-Acetoxy-3-oxa-bicyclo[4.3.0]non-1-en-9-on.

5. Verfahren zur Herstellung der Verbindungen der allgemeinen Formel I, dadurch gekennzeichnet, daß man

(a) Catalpol der Formel II

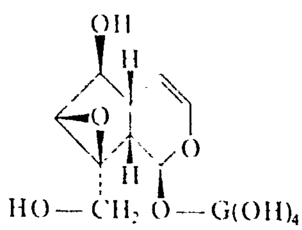

(II)

—O—G(OH)₄ = 1-β-D-Glucopyranosyl-Rest

das aus Picrorhiza kurrooa gewonnen werden kann, mit dem Anhydrid einer Carbonsäure mit 2 bis 6 C-Atomen in einem geeigneten Lösungsmittel zum entsprechenden Hexaacylcatalpol acyliert (O-Acylierung),

(b) das Hexaacyl-catalpol durch katalytische Hydrierung in das entsprechende Hexaacyl-dihydrocatalpol überführt,

(c) das Hexaacyl-dihydrocatalpol durch regioselektive Epoxidaufstellung mittels Lithiumalanat-Reduktion zu 6,8-Dihydroxy-8-hydroxymethyl-1-iridanyl-1′-β-D-glucopyranosid umsetzt und

(d) letzteres mit Perjodsäure oder einem ihrer Salze oder mit Bleitetraacetat oxidiert und die Reaktionslösung mit einer schwachen Base sättigt,
(e) wonach gegebenenfalls die Hydroxylgruppe in 6-Stellung des Iridoid-Gerüstes nach an sich bekannten Methoden alkyliert oder acyliert oder kondensiert wird.

6. Verfahren nach Anspruch 5, dadurch gekennzeichnet, daß die Lithiumalanat-Reduktion in einem dipolaren aprotischen Lösungsmittel, vorzugsweise in absolutem Tetrahydrofuran, durchgeführt wird.
7. Verwendung der Verbindungen gemäß einem der Ansprüche 1 bis 4 als Zwischenprodukte zur Herstellung von Prostanoiden.

**Claims**

1. Reaktive derivatives of 3-oxa-bicyclo[4.3.0]non-1-en-9-one of the general formula I,

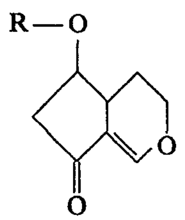

(I)

wherein R is a hydrogen atom, an alkyl group with 1 to 5 C-atoms, an acyl group with 2 to 6 C-atoms, a non-substituted aralkyl group with 7 to 12 C-atoms, a methanesulfonyl- or toluenesulfonyl- group, a benzoyl-, nitrobenzoyl- or chlorobenzoyl- or a tetrahydropyranyl- group.
2. 7-Hydroxy-3-oxa-bicyclo[4.3.0]non-1-en-9-one.
3. (6R, 7R)-(—)-7-Hydroxy-3-oxa-bicyclo[4.3.0]non-1-en-9-one.
4. 7-Acetoxy-3-oxa-bicyclo[4.3.0]non-1-en-9-one.
5. Process for the manufacture of the compounds of general formula I, characterized in that

a) Catalpol of the formula II

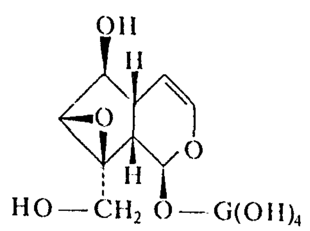

(II)

(—O—G(OH)$_4$ = 1-β-D-Glucopyranosyl)

which can be extracted from Picrorhiza kurrooa, is acylated to form the corresponding hexaacyl-catalpol (O-acylation) with the anhydride of a carboxylic acid with 2 to to 6 C-atoms in a suitable solvent,
b) the hexaacyl-catalpol is converted to the corresponding hexaacyl-dihydrocatalpol by means of catalytic hydrogenation,
c) the hexaacyl-dihydrocatalpol is converted to 6,8-dihydroxy-8-hydroxymethyl-1-iridanyl-1'-β-D-glucopyranoside via regioselective epoxide splitting by means of lithium aluminium hydride reduction,
d) the substance thus produced then being oxidized with periodic acid or one of its salts or with lead tetraacetate, and the reaction solution then being saturated with a weak base,
e) after which, optionally, the hydroxyl group at position 6 of the iridoid skeleton is alkylated or acylated or condensed according to methods known per se.

6. Process according to claim 5, characterized in that the lithium aluminium hydride reduction is performed in a dipolar aprotic solvent, preferably in absolute tetrahydrofuran.
7. Use of the compounds according to any one of claims 1 to 4 as intermediates for the manufacture of prostanoids.

**Revendications**

1. Dérivés réactifs de la 3-oxa-bicyclo[4.3.0]non-1-én-9-one de formule générale I

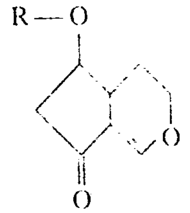

(I)

où R représente un atome d'hydrogène, un groupe alcoyle en $C_1$ à $C_5$, un groupe acyle en $C_2$ à $C_6$, un groupe aralcoyle non substitué en $C_7$ à $C_{12}$, un groupe méthanesulfonyle ou un groupe toluènesulfonyle, un groupe benzoyle, nitrobenzoyle ou chlorobenzoyle ou un groupe tétrahydropyranyle.

2. La 7-hydroxy-3-oxa-bicyclo[4.3.0]non-1-én-9-one.

3. La (6R, 7R)-(−)-7-hydroxy-3-oxa-bicyclo[4.3.0]non-1-én-9-one.

4. La 7-acétoxy-3-oxa-bicyclo[4.3.0]non-1-én-9-one.

5. Procédé de préparation des composés de formule générale I, caractérisé en ce que

(a) on acyle du Catalpol de formule II

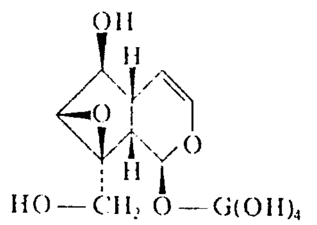

(II)

$-O-H(OH)_4$ = radical 1-β-D-glucopyranosyle,

que l'on peut obtenir à partir de Picrorhiza kurrooa, avec l'anhydride d'un acide carboxylique en $C_2$ à $C_6$ dans un solvant approprié pour donner l'hexaacyl-catalpol correspondant (O-acylation),

(b) on transforme l'hexaacyl-catalpol par hydrogénation catalytique pour donner l'hexaacyl-dihydrocatalpol correspondant,

(c) on fait réagir l'hexaacyl-dihydrocatalpol par un clivage époxyde régio-sélectif au moyen d'une réduction à l'alanate de lithium pour donner le 6,8-dihydroxy-8-hydroxyméthyl-1-iridanyl-1'-β-D-glucopyranoside et

(d) on oxyde ce dernier avec de l'acide periodique ou un de ses sels ou avec du tétraacétate de plomb et on sature la solution réactionnelle avec une base faible,

(e) après quoi on alcoyle, on acyle ou on condense le cas échéant le groupe hydroxyle en position 6 du squelette iridoïde selon des procédés connus.

6. Procédé selon la revendication 5, caractérisé en ce qu'on conduit la réduction à l'alanate de lithium dans un solvant aprotique dipolaire, de préférence dans le tétrahydrofuranne absolu.

7. Application des composés selon l'une des revendications 1 à 4 comme produits intermédiaires pour la préparation de prostanoïdes.